(19) Europäisches Patentamt
European Patent Office
Office européen des brevets

(11) **EP 4 478 257 A1**

(12) **EUROPEAN PATENT APPLICATION**

(43) Date of publication:
**18.12.2024 Bulletin 2024/51**

(21) Application number: **23305965.8**

(22) Date of filing: **16.06.2023**

(51) International Patent Classification (IPC):
**G06N 3/088** $^{(2023.01)}$ **G06N 3/0895** $^{(2023.01)}$
**G06N 20/00** $^{(2019.01)}$ **A61N 5/10** $^{(2006.01)}$

(52) Cooperative Patent Classification (CPC):
**A61N 5/1031; G06N 3/0464; G06N 3/0475;**
**G06N 3/088; G06N 3/09; G06N 3/0985;**
A61N 2005/1041; G06N 3/084; G06N 3/0895

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicants:
• **TheraPanacea**
 **75004 Paris (FR)**
• **Institut de Cancerologie Gustave Roussy**
 **94805 Villejuif Cedex (FR)**
• **CentraleSupélec**
 **91190 Gif-sur-Yvette (FR)**

(72) Inventors:
• **MARTINOT, Sonia**
 **75004 PARIS (FR)**
• **PARAGIOS, Nikos**
 **75004 PARIS (FR)**
• **VAKALOPOULOU, Maria**
 **91190 GIF-sur -YVETTE (FR)**
• **ROBERT, Charlotte**
 **94805 VILLEJUIF Cedex (FR)**
• **DEUTCH, Eric**
 **94805 VILLEJUIF Cedex (FR)**

(74) Representative: **Icosa**
 **83 avenue Denfert-Rochereau**
 **75014 Paris (FR)**

(54) **DEVICE FOR OBTAINING A TRAINED AUGMENTED RADIOTHERAPY GENERATED MODEL**

(57) The present invention relates to a device for obtaining a trained augmented radiotherapy generative model, configured to generate augmented radiotherapy dose distributions from input, previously calculated, radiotherapy dose distributions, said device comprising:
- at least one input configured to receive a training data set comprising at least one training sample,
- at least one processor configured to define said augmented radiotherapy generative model by training an untrained generative model using said training dataset and a GPR loss function,
- at least one output configured to provide as output said trained augmented radiotherapy generative model.

FIG. 1

EP 4 478 257 A1

**Description**

**FIELD OF INVENTION**

The present invention relates to dose modeling in the context of photon radiation therapy.

More specifically, the invention relates to a device for obtaining a trained augmented radiotherapy generative model and to a device for generating augmented radiotherapy dose distributions from input previously calculated radiotherapy dose distributions, using the trained augmented radiotherapy generative model.

**BACKGROUND OF INVENTION**

In photon radiation therapy, accurate dose modeling is paramount to ensure treatment plans safely target the tumor. Monte Carlo simulation is considered the gold standard for accuracy in radiotherapy dose calculations due to its detailed modeling of individual physical interaction processes. Yet, Monte Carlo generation of radiotherapy dose distributions remains too time-consuming for clinical adoption. Recent deep learning accelerated Monte Carlo dose calculation methods offer a solution for this problem, utilizing common computer vision loss functions. Even if such methods provide a good trade-off between time and performance, training on such loss functions amounts to solving a proxy problem, with no strict assurance to conjointly optimize the clinical validation of the dose generated by those methods.

Indeed, it is of primary importance to evaluate and verify the accuracy and quality of a calculated dose distribution in relation to the intended treatment plan for a patient. More specifically, during the treatment planning phase in radiotherapy, experts design a treatment plan that optimally delivers the prescribed radiation dose to the tumor while minimizing the dose to surrounding healthy tissues. For instance, clinical validation of calculated dose involves the validation of the dose calculation accuracy, the evaluation of dose constraints including maximum allowable dose values to critical structures and minimum required dose value to the tumor, a general plan evaluation including evaluation of dose coverage, dose homogeneity, and adherence to dose constraints.

The present invention aims at providing a solution to compute dose distributions in an accurate while fast manner while avoiding facing a proxy problem as described above.

**SUMMARY**

This invention thus relates to a device for obtaining a trained augmented radiotherapy generative model, configured to generate augmented radiotherapy dose distributions from input, previously calculated, radiotherapy dose distributions, said device comprising:

- at least one input configured to receive a training data set comprising at least one training sample, each training sample comprising at least one radiotherapy dose distribution defined on a domain discretized into a plurality of individual elements
- at least one processor configured to define said augmented radiotherapy generative model by training an untrained generative model using said training dataset and a GPR loss function,
  wherein said GPR loss function is defined based on a differentiable function approximating a clinical similarity metric, being a Gamma index Passing Rate (GPR), said GPR measuring a similarity between a target radiotherapy dose distribution and a calculated radiotherapy dose distribution,
- at least one output configured to provide as output said trained augmented radiotherapy generative model.

Thus, advantageously, the device according to the invention integrates a mathematical objective that trains a generative model to optimize a clinically relevant property, therefore leading to a more accurate training from a clinical standpoint. Besides, since a differentiable function is used as a loss function, non-null gradients can be obtained and used in, for instance, a gradient descent algorithm (i.e., preventing any problem of gradient vanishing).

In some embodiments, the domain is $\mathbb{R}^k$, with k an integer number equal to 1, 2 or 3. Therefore, the device according to the invention can be used in the cased of one-dimensional dose distributions, 2D dose distributions or 3D dose distributions.

In some embodiments, the clinical similarity metric is the gamma index passing rate, corresponding to a proportion of said individual elements of said domain satisfying a passing criterion, wherein said passing criterion is based, for each individual element, on a gamma index value.

In some embodiments, the differentiable function is defined by means of a sum of sigmoid functions of sharpness parameter $\beta$.

In some embodiments, the at least one processor is further configured to vary, during training, said parameter $\beta$. Notably said parameter $\beta$ is varied by implementing an annealing schedule. In this manner, the optimization performed during a training process with the device according to the invention can be adjusted and assisted with such an annealing schedule.

In some embodiments, the annealing schedule comprises increasing said parameter $\beta$ during training, and said at least one processor is further configured to, above a given threshold value of said parameter $\beta$, apply a stopgrad operation for individual elements not satisfying said passing criterion. Indeed, at the beginning of a training process with the device according to the invention, the model predicts poorly and the majority of the individual elements of the domain fail to pass the passing criterion, meaning that the approximation function generates zero gradients. Therefore, starting the training with low values of the parameter $\beta$ helps initiating the optimization process. Then, the parameter $\beta$ is increased so that the sigmoid functions are better adapted to approximate an ideal non-differentiable GPR-based loss function, therefore using more accurately the Gamma index Passing Rate metric in the computation of the GPR loss function. As the parameter $\beta$ increases, zero gradients may be backpropagated, since the sigmoid functions resemble more and more to indicator functions, which generate zero gradients. Therefore, advantageously, the application of a stopgrad function allows preventing the computation of such zero gradients and, as a consequence, their backpropagation.

In some embodiments, the at least one processor is further configured to compute a plurality of gamma index values corresponding to each individual element of said domain, all among the plurality of gamma index values being computed by means of a minimum value search within a vicinity of invariant size. Those embodiments allow to accelerate the computation of the gamma index values by limiting a sub-domain where a minimum search value is performed to compute the gamma index values, and thus, the computation of the GPR loss function is accelerated as a consequence during the training process.

In some embodiments, the vicinity is a cube comprising $(2 \times DTA +1)^k$ voxels.

In some embodiments, the at least one generative model is a U-Net.

In some embodiments, each training sample comprises at least two radiotherapy dose distributions, a first reference radiotherapy dose distribution, defined on said domain, and a corresponding second reference radiotherapy dose distribution, also defined on said domain; wherein each first reference radiotherapy dose distribution has higher quality than said corresponding second reference radiotherapy dose distribution. According to those embodiments, the at least one processor is configured to obtain the trained augmented generative model in a supervised manner.

Therefore, the obtained trained augmented radiotherapy generative model is trained to generate high quality dose distributions from inputted low quality dose distributions. In other words, the obtained trained augmented radiotherapy generative model is configured to generate accurate dose distributions from rough dose distributions, for instance dose distributions obtained from quick (i.e., low statistics) and approximative dose distributions.

In some embodiments, for each training sample, said first reference radiotherapy dose distribution has been generated from a Monte-Carlo algorithm with a number of simulated particles having order of magnitude at least one time superior than said corresponding second reference radiotherapy dose distribution, also obtained from the same Monte-Carlo algorithm.

In some embodiments, for each training sample, said first reference radiotherapy dose distribution is obtained from a Monte Carlo algorithm and said corresponding second radiotherapy dose distribution is obtained from a collapse-cone convolution algorithm.

Therefore, the obtained trained augmented radiotherapy generative model is trained to generate Monte Carlo precision-like dose distributions from low precision dose distributions obtained by a collapse-cone convolution algorithm.

In some embodiments:

- each training sample comprises a first reference radiotherapy dose distribution defined on said domain and a corresponding second reference radiotherapy dose distribution also defined on said domain, wherein each first reference radiotherapy dose distribution and each corresponding second reference radiotherapy dose distribution have low quality;
- said at least one processor is configured to define said augmented radiotherapy generative model by training said untrained generative model using said training dataset and the GPR loss function in a weakly-supervised manner.

In some other embodiments, the at least one processor is configured to define said augmented radiotherapy generative model by training said untrained generative model using said training dataset and the GPR loss function in an unsupervised manner.

Another aspect of the invention relates to a device for generating augmented radiotherapy dose distributions from input previously calculated radiotherapy dose distributions, using the augmented radiotherapy generative model obtained from the device for obtaining a trained augmented radiotherapy generative model previously described, said device comprising:

- at least one input configured to receive at least one input previously calculated radiotherapy dose distribution,
- at least one processor configured to provide said at least one input previously calculated radiotherapy dose distribution to said augmented radiotherapy generative model so as to obtain at least one augmented radiotherapy dose distribution,
- at least one output configured to provide said augmented radiotherapy dose distributions.

Therefore, advantageously, the device according to the invention allows obtaining high quality dose distributions from low quality dose distributions. For instance, the device according to the invention allows obtaining high quality dose distributions from dose distributions obtained from quick and approximative calculations. The device according to the invention therefore allows for acceleration of the obtention of high quality dose distributions.

In some embodiments, the input previously calculated radiotherapy dose distributions are obtained from a Monte Carlo algorithm or from a collapse-cone convolution algorithm, and said augmented radiotherapy dose distributions present higher resolution than said input previously calculated radiotherapy dose distributions. Therefore, the device according to the invention allows for super-resolution achievement.

In addition, the disclosure relates to a computer program comprising software code adapted to perform a method for generating augmented radiotherapy dose distributions or a method for training compliant with any of the above execution modes when the program is executed by a processor.

The present disclosure further pertains to a non-transitory program storage device, readable by a computer, tangibly embodying a program of instructions executable by the computer to perform a method for generating augmented radiotherapy dose distributions or a method for training, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

DEFINITIONS

In the present invention, the following terms have the following meanings:

The terms "adapted" and "configured" are used in the present disclosure as broadly encompassing initial configuration, later adaptation or complementation of the present device, or any combination thereof alike, whether effected through material or software means (including firmware).

The term "**processor**" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

"**Machine learning (ML)**" designates in a traditional way computer algorithms improving automatically through experience, on the ground of training data enabling to adjust parameters of computer models through gap reductions between expected outputs extracted from the training data and evaluated outputs computed by the computer models.

"**Datasets**" are collections of data used to build an ML mathematical model, so as to make data-driven predictions or decisions. In "**supervised learning**" (i.e. inferring functions from known input-output examples in the form of labelled training data), three types of ML datasets (also designated as ML sets) are typically dedicated to three respective kinds of tasks: "**training**", i.e. fitting the parameters, "**validation**", i.e. tuning ML hyperparameters (which are parameters used to control the learning process), and "**testing**", i.e. checking independently of a training dataset exploited for building a mathematical model that the latter model provides satisfying results.

A "**neural network (NN)**" designates a category of ML comprising nodes (called "**neurons**"), and connections between neurons modeled by "**weights**". For each neuron, an output is given in function of an input or a set of inputs by an "**activation function**". Neurons are generally organized into multiple "**layers**", so that neurons of one layer connect only to neurons of the immediately preceding and immediately following layers.

The above ML definitions are compliant with their usual meaning, and can be completed with numerous associated features and properties, and definitions of related numerical objects, well known to a person skilled in the ML field. Additional terms will be defined, specified or commented wherever useful throughout the following description.

## BRIEF DESCRIPTION OF THE DRAWINGS

The present disclosure will be better understood, and other specific features and advantages will emerge upon reading the following description of particular and non-restrictive illustrative embodiments, the description making reference to the annexed drawings wherein:

**Figure 1** is a block diagram representing schematically a particular mode of a device for obtaining an augmented radiotherapy generative model, compliant with the present disclosure.

**Figure 2** is a flow chart showing successive steps executed with the device for obtaining an augmented radiotherapy generative model of figure 1.

**Figure 3** is a block diagram representing schematically a particular mode of a device for generating augmented radiotherapy dose distributions from input, previously calculated radiotherapy dose distributions, using an augmented radiotherapy generative model obtained from the device represented in Figure 1, compliant with the present disclosure;

**Figure 4** is a flow chart showing successive steps executed with the device for generating augmented radiotherapy dose distributions from input, previously calculated radiotherapy dose distributions of figure 3;

**Figures 5A-5D** show single slices corresponding to respectively a $1e^9$ dose volume, a prediction of a model trained with MSE, a prediction of a model trained with $L_{\sigma-GPR}^{\delta}$ and a reference $1e^{11}$ dose; **Figures 5E-5G** show corresponding gamma index maps of **Figures 5A-5C.**

**Figure 6** shows execution times for an SSIM-based computation method, an accelerated computation method according to the present disclosure and a reference exhaustive search-based method;

**Figures 7A-7B** show respectively a dose prediction resulting from the CCC to MC translation and a reference MC dose distribution; **Figure 7C** shows the gamma index map corresponding to the dose distribution of **Figure 7A**.

## DETAILED DESCRIPTION

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

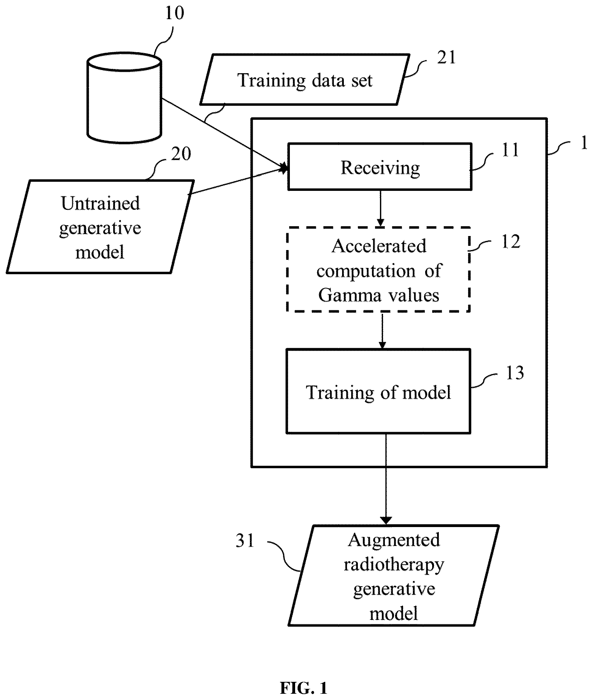

The present disclosure will be described in reference to a particular functional embodiment of a device 1 for obtaining a trained augmented radiotherapy generative model, configured to generate augmented radiotherapy dose distributions from input, previously calculated, radiotherapy dose distributions, as illustrated on Figure 1.

The device 1 is adapted to produce a trained generative model, that in the present disclosure is called augmented radiotherapy generative model 31, which is configured to receive as an input, previously calculated, radiotherapy dose distributions 32, and generate as output augmented radiotherapy dose distributions 41. By augmented radiotherapy dose distributions it has to be understood a radiotherapy dose distribution that respects the clinical standards. Notably, the device 1 is configured to train a generative (learning) model using a training data set 21 comprising at least one training sample in order to obtain the trained augmented radiotherapy generative model 31. As will be seen further, the trained augmented radiotherapy generative model 31 is configured to generate in an accelerated and accurate manner augmented radiotherapy dose distributions 41 based on low precision input dose distributions quickly obtained from previous computations based on treatment planning data such as optimal radiation beam arrangements, beam energies, and a 3D image of the patient. For instance, the 3D image of the patient is a CT scan.

The input data for the device 1 may be the training data set 21 and the untrained architecture of the generative model 20.

In the present disclosure, the training data set 21 comprises at least one training sample. Each training sample comprises at least one radiotherapy dose distribution defined on a domain discretized into a plurality of individual elements.

In some embodiments, the domain is $\mathbb{R}^k$, with k an integer number equal to 1, 2 or 3. In other words, the at least one radiotherapy dose distribution can be a one-dimensional vector, where each element of the vector is assigned a dose value, a bidimensional matrix with pixels, where each pixel is assigned a dose value, or a 3D volume with voxels, where each voxel is assigned a dose value.

In some embodiments, each training sample comprises at least two radiotherapy dose distributions, a first reference radiotherapy dose distribution, defined on the domain, and a corresponding second reference radiotherapy dose distribution, also defined on the same domain.

Advantageously, in those embodiments, the first reference radiotherapy dose distribution is used as a ground truth and the second reference radiotherapy dose distribution serves as an input for the augmented radiotherapy generative model that is to be trained by the device 1.

In those embodiments comprising these training samples with two radiotherapy dose distributions, referred to as supervised embodiments, the device 1 is configured to obtain the trained augmented radiotherapy generative model 31 in a supervised manner.

For instance, each first reference radiotherapy dose distribution (i.e., ground truth) has higher quality than said corresponding second reference radiotherapy dose distribution. In a first example, the first reference radiotherapy dose distribution and the second reference radiotherapy dose distribution have been obtained from a Monte Carlo algorithm. For instance, the first reference radiotherapy dose distribution has been generated with a number of simulated particles having order of magnitude at least one time superior than said corresponding second reference radiotherapy dose distribution. In a second example, the first reference radiotherapy dose distribution is obtained from a Monte Carlo algorithm and said corresponding second radiotherapy dose distribution is obtained from a collapse-cone convolution algorithm.

In those embodiments, a first reference radiotherapy dose distribution of given high quality can be associated to a plurality of second reference radiotherapy dose distributions, leading to a plurality of corresponding training samples.

In some embodiments referred to as weakly supervised embodiments, the device 1 is configured to obtain the trained augmented radiotherapy generative model 31 in a weakly supervised manner. For instance, the first reference radiotherapy dose distribution and the corresponding second reference radiotherapy dose distribution of one training sample have both low quality. For instance, they are obtained from a Monte Carlo algorithm implemented with a comparable number of simulated particles. In another example, they present the same spatial resolution.

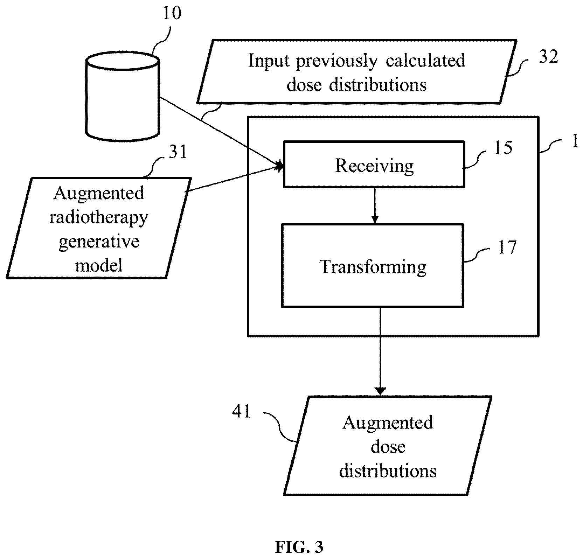

The device 1 for obtaining the trained augmented radiotherapy generative model 31 (i.e., trained so as to set the augmented radiotherapy generative model parameters) is associated with a device 2, represented on Figure 3, for generating augmented radiotherapy dose distributions 41 from inputted, previously calculated, radiotherapy dose distributions 32, using the trained augmented radiotherapy generative model 31 obtained from the device 1, which will be subsequently described.

Though the presently described devices 1 and 2 are versatile and provided with several functions that can be carried out alternatively or in any cumulative way, other implementations within the scope of the present disclosure include devices having only parts of the present functionalities.

Each of the devices 1 and 2 is advantageously an apparatus, or a physical part of an apparatus, designed, configured and/or adapted for performing the mentioned functions and produce the mentioned effects or results. In alternative implementations, any of the device 1 and the device 2 is embodied as a set of apparatus or physical parts of apparatus, whether grouped in a same machine or in different, possibly remote, machines. The device 1 and/or the device 2 may e.g. have functions distributed over a cloud infrastructure and be available to users as a cloud-based service, or have remote functions accessible through an API.

The device 1 for training and the device 2 for generating augmented radiotherapy dose distributions 41 may be integrated in a same apparatus or set of apparatus, and intended to same users. In other implementations, the structure of the device 2 may be completely independent of the structure of the device 1, and may be provided for other users. For example, the device 2 may have a parameterized generative model available to operators for augmented radiotherapy dose distribution generation, wholly set from previous training effected upstream by other players with the device 1.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the device 1 or of the device 2.

The device 1 comprises a module 11 for receiving the training data set 21 and the untrained generative model 20, stored in one or more local or remote database(s). The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

The device 1 further comprises a module 13 configured to train the generative model 20 using the training data set 21 received by the module 11.

The generative (learning) model, trained in module 13, is a neural network. In one example, the generative (learning) model, is based on an architecture of a U-Net model.

As will be explained below, the module 13 trains the generative model using a loss function referred to as GPR loss function, as it is defined based on the Gamma index Passing Rate metric. The Gamma index Passing Rate metric (GPR) is a clinical similarity metric, that measures a similarity between a target radiotherapy dose distribution and a calculated radiotherapy dose distribution. The Gamma index Passing Rate is considered the preferred metric to evaluate dose distributions in order to deliver sage radiotherapy treatments. The GPR is one of the most essential and commonly used clinical evaluation metric for verification of complex radiotherapy dose delivery such as Intensity Modulated Radiation Therapy or Volumetric Modulated Arc Therapy (VMAT). As such, the GPR provides a clinical criterion to assess the quality of a model's prediction.

The Gamma index Passing Rate is based on the Gamma index value defined as below.

Dr and De are two dose distributions defined on $\mathbb{R}^k$, with k an integer number equal to 1, 2 or 3, and having values in R. Dr is a reference distribution and De is an evaluated distribution calculated so as to be as similar as possible to the reference distribution Dr. The domain $\mathbb{R}^k$ is discretized into a plurality of individual elements. For instance, if k is equal to 2, the individual elements are pixels. If k is equal to 3 the individual elements are voxels. Each point Pr on which the reference distribution Dr is defined has a coordinate vector $\vec{d}(P_r)$ and a corresponding dose value Dr(Pr). Similarly, each point Pe on which the evaluated distribution De is defined has a coordinate vector $\vec{d}(P_e)$ and a corresponding dose value De(Pe). The Gamma index value for a point Pr where the reference dose distribution is defined, given a vicinity V(Pr) comprising a set of points Pe around Pr, is defined by:

$$\Gamma(P_r) = \min_{P_e \in V(P_r)} \sqrt{\frac{\left\| \vec{d}(P_e) - \vec{d}(P_r) \right\|^2}{DTA^2} + \frac{\left( D_e(P_e) - D_r(P_r) \right)^2}{\Delta^2}} \qquad (1)$$

where DTA designates the tolerance on the Distance-To-Agreement, commonly in mm and $\Delta$ designates the tolerance on the relative dose difference expressed as a percentage of the reference dose value Dr(Pr).

Therefore, a Gamma index value is defined for each point Pr on which the reference dose distribution and this value indicates how close neighboring points Pe of Pr are, both spatially and dose-wise.

The Gamma index Passing Rate GPR is related to the Gamma index value and to a passing criterion.

A dose threshold $\delta$ is considered, as well as a point Pr where the reference distribution Dr is defined and for which a threshold criterion is satisfied:

$$\mathrm{Dr(Pr)} > \delta \qquad (2).$$

Given a tolerance on the Distance-To-Agreement DTA and a dose tolerance $\Delta$, the passing criterion is defined by:

$$\Gamma(P_r) \leq 1 \qquad (3)$$

The GPR is defined as the percentage of points Pr satisfying the passing criterion (3) while satisfying the threshold condition (2). The GPR can be written as:

$$GPR(D_r, D_e) = \frac{\sum_{P_r \in D_r} 1_{D_r \geq \delta}(P_r) \cdot 1_{\Gamma \leq 1}(P_r)}{\sum_{P_r \in D_r} 1_{D_r \geq \delta}(P_r)} \qquad (4)$$

where $1_{D_r \geq \delta}$ and $1_{\Gamma \leq 1}$ are indicator functions defined such that:

$$1_{D_r \geq \delta}(P_r) = \begin{cases} 1 & if\ D_r(P_r) \geq \delta \\ 0 & otherwise \end{cases} \text{ and } 1_{\Gamma \leq 1}(P_r) = \begin{cases} 1 & if\ \Gamma(P_r) \leq 1 \\ 0 & otherwise \end{cases}$$

Equation (4) shows that the GPR takes values between 0 and 1. The evaluated distribution De approaches the reference distribution Dr as the GPR increases towards 1.

Therefore, a preliminary loss function can be defined as:

$$L^\delta_{GPR} = 1 - GPR(D_r, D_e) = 1 - \frac{\sum_{P_r \in D_r} 1_{D_r \geq \delta}(P_r) \cdot 1_{\Gamma \leq 1}(P_r)}{\sum_{P_r \in D_r} 1_{D_r \geq \delta}(P_r)} \qquad (5)$$

It can be noticed that the preliminary loss function $L^\delta_{GPR}$ is comprised between 0 and 1 and decreases towards 0 as the GPR increases towards 1. Therefore, the preliminary loss function could serve as a suitable starting point for defining the GPR loss function to be used by the module 12 to train the generative model. Indeed, trying to minimize the GPR loss function and making it approaching 0 would meanwhile optimize the GPR towards values close to 1. This observation converges with the principle according to which integrating mathematical objectives that train machine learning models to optimize clinically relevant properties is of utmost interest for their integration into clinical practice.

However, the preliminary loss function $L^\delta_{GPR}$ is not differentiable due to the presence of the indicator functions, which are stepwise and generate null gradients. As a consequence, the preliminary loss function $L^\delta_{GPR}$ is not suitable, as is, for algorithms used during training processes such as gradient descent algorithms. It can be intuited that, if found, the use of a differentiable loss function based on the preliminary loss function $L^\delta_{GPR}$ would yield to more accurate training from a clinical standpoint.

The inventors of the present invention overcame the mathematical challenge of the GPR and found a way to turn this clinical metric into a viable loss function for the task of accelerating the simulation of radiotherapy dose distributions. More specifically, they found a suitable way to approximate the preliminary loss function $L^\delta_{GPR}$ by using differentiable functions.

The approximation uses the sigmoid function:

$$\sigma(x) = \left(1 + exp(-\beta x)\right)^{-1}$$

where β represents a sharpness parameter. More specifically, β represents the slope of the sigmoid function at x equal to 0.

It can be noticed that:

$$\lim_{\beta \to +\infty} \sigma\left(\beta\left(1 - \Gamma(P_r)\right)\right) = 1_{\Gamma \leq 1}(P_r)$$

Therefore, the GPR loss function can be defined as:

$$L^\delta_{\sigma-GPR} = 1 - \frac{\sum_{P_r \in D_r} \sigma\left(\beta\left(1 - \Gamma(P_r)\right)\right) . 1_{D_r \geq \delta}(P_r)}{\sum_{P_r \in D_r} 1_{D_r \geq \delta}(P_r)} \qquad (6)$$

The GPR loss function as defined in Equation (6) is defined by means of a sum of sigmoid functions of sharpness parameter β and is thus differentiable and, provided the sharpness parameter β is not too high, allows gradient descent during a training process aiming at updating weights of a neural network-based generative model during backpropagation.

The module 12 is further configured to perform optimization of the generative learning model 20 using a stochastic gradient descent algorithm such as for example an Adam solver algorithm, Stochastic Gradient Descendent (SGD), AdaGrad algorithm, or the like.

In some embodiments, the module 13 is configured to perform an annealing schedule on the sharpness parameter β during the optimization of the generative learning model. An example of the annealing schedule, where the annealing schedule starts with low initial values of β and progressively increases β over the training phase is described below.

For instance, at the beginning of the training, an approximation of the GPR loss function can be used, and is defined by:

$$L^\delta_\Gamma(D_r, D_e) = \frac{\sum_{P_r \in D_r} \Gamma(P_r) . 1_{D_r \geq \delta}(P_r)}{\sum_{P_r \in D_r} 1_{D_r \geq \delta}(P_r)} \qquad (7)$$

This approximation is obtained by using the Taylor expansion of the sigmoid function and the resulting Taylor expansion of the GPR loss function, when $\beta \approx 0^+$:

$$\sigma\left(\beta\left(1 - \Gamma(P_r)\right)\right) \approx \beta\left(1 - \Gamma(P_r)\right)$$

$$L^\delta_{\sigma-GPR}(D_r, D_e) \approx 1 - \beta + \frac{\sum_{P_r \in D_r} \Gamma(P_r) . 1_{D_r \geq \delta}(P_r)}{\sum_{P_r \in D_r} 1_{D_r \geq \delta}(P_r)}$$

As the training continues, and the approximation $L^\delta_\Gamma(D_r, D_e)$ given in Equation (7) of $L^\delta_{\sigma-GPR}(D_r, D_e)$ decreases, the module 13 is configured to proceed with the annealing schedule in progressively increasing β so as to improve the approximation $L^\delta_{\sigma-GPR}$ given in Equation(6) of $L^\delta_{GPR}$. As β increases and is acquiring larger values, minimizing the loss function $L^\delta_{\sigma-GPR}$ amounts to get individual elements of the domain not passing the passing criterion defined by the inequation (3) to pass the passing criterion.

To model the behavior of $L^\delta_{\sigma-GPR}$ at this stage, i.e. as β is acquiring larger values and exceeds a given threshold, the module 13 is configured to optimize the generative learning model by using a modified loss function defined as:

$$L^\delta_{\Gamma>1}(D_r, D_e) = \frac{\sum_{P_r \in D_r} \Gamma(P_r) . 1_{D_r \geq \delta}(P_r) . stopgrad\left(1_{\Gamma>1}(P_r)\right)}{\sum_{P_r \in D_r} 1_{D_r \geq \delta}(P_r)} \qquad (8)$$

where the *stopgrad(.)* function is a function that prevents from computing the gradient for given variables and

$$1_{\Gamma>1}(P_r) = \begin{cases} 1 & if\ \Gamma(P_r) > 1 \\ 0 & otherwise \end{cases}.$$

The modified loss function $L^\delta_{\Gamma>1}(D_r, D_e)$ is meant to get failing voxels (i.e., voxels - individual elements- with Γ > 1) to satisfy the passing criterion.

It can be inferred from the expression of the GPR loss function $L^{\delta}_{\sigma-GPR}$ defined in Equation (6) and of the its

different approximations $L^{\delta}_{\Gamma}(D_r, D_e), L^{\delta}_{\Gamma>1}(D_r, D_e)$ defined in Equations (7) and (8) used during the training, that the computation of those functions requires computing the Gamma index value over all individual elements of the domain.

Thus, in some embodiments, referred to as acceleration embodiments, the device 1 further comprises a module 12 configured, for the computation of the loss function, to compute the Gamma index value as defined in Equation (1) by means of a minimum value search within a vicinity of invariant size. More specifically, the vicinity of invariant size is defined by the chosen tolerance on the Distance-To-Agreement (DTA). For instance, the vicinity of a point Pr of the domain $\mathbb{R}^k$ is a cube comprising $(2 \times DA+1)^k$ individual elements. The use of this vicinity of invariant size enables fast computation of the Gamma index values over the domain $\mathbb{R}^k$.

Therefore, once the training is completed, the module 13 is configured to output the trained augmented radiotherapy generative model 31. The augmented radiotherapy generative model 31 may then by stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

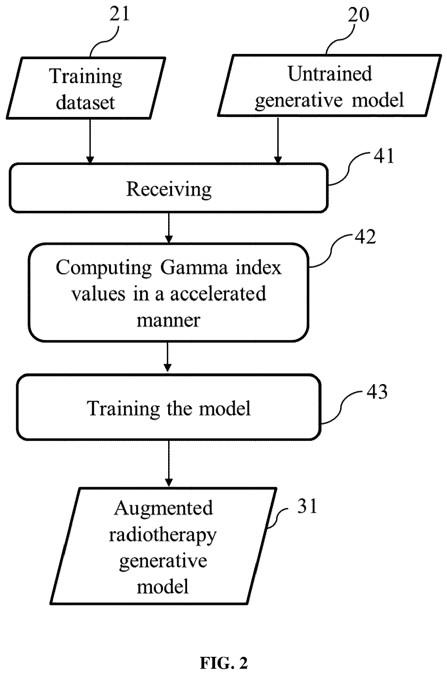

In its automatic actions, the device 1 may for example execute the following process (Figure 2):

- receiving the training data set (step 41),
- optionally compute the Gamma index values in an accelerated manner (step 42)
- training the generative model 20 using the training data set and the GPR loss function previously described (step 43).

The present invention also relates to a device 2 for generating augmented radiotherapy dose distributions 41, using the augmented radiotherapy generative model 31 obtained from the device 1, as described above. The device 2 will be described in reference to a particular function embodiment as illustrated in Figure 3.

The device 2 is adapted to receive as input the augmented CT generative model 31, already trained, and at least one input previously calculated radiotherapy dose distribution 32. For instance, the input previously calculated radiotherapy dose distributions 32 are low precision input dose distributions quickly obtained from previous computations based on treatment planning data such as optimal radiation beam arrangements, beam energies, and 3D images of a patient. For instance, the input previously calculated radiotherapy dose distributions are obtained with a collapse-cone convolution algorithm, or present a low spatial resolution. For instance also, 3D images of a patient can be CT scans or measurements of electron density. The device 2 is adapted to provide as output augmented radiotherapy dose distributions 41 obtained from the transformation of the input previously calculated radiotherapy dose distributions 32 performed by the augmented radiotherapy generative model 31. Therefore, the device 2 is configured to transform one low quality dose distribution into one augmented radiotherapy dose distribution, representing the same dose distribution of the input dose distribution, but having improved quality.

The device 2 comprises a module 15 for receiving the augmented radiotherapy generative model 31 and the at least one input previously calculated dose distribution 32, stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk). In advantageous embodiments, the augmented radiotherapy generative model 31 and all its parameters have been previously generated by a system including the device 2 for training. Alternatively, the augmented radiotherapy generative model 31 and its parameters are received from a communication network.

The device 2 further comprises a module 17 configured to provide the input dose distribution 32 to said augmented radiotherapy generative model 31 so as to generate a corresponding augmented dose distribution 41 with improved quality.

The device 2 may interact with a user interface 18, via which information can be entered and retrieved by a user. The user interface 18 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

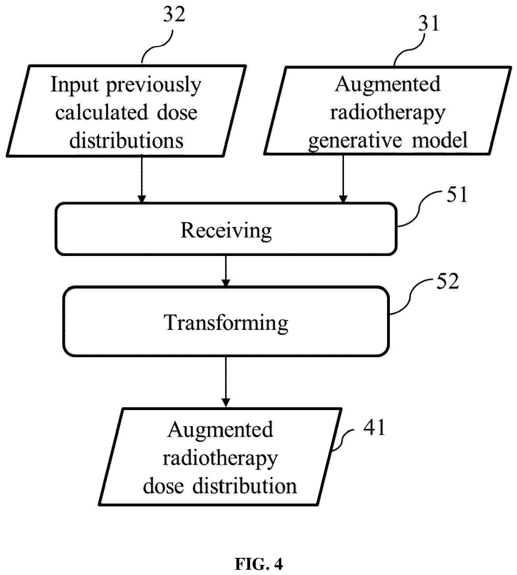

In its automatic actions, the device 2 may for example execute the following process (Figure 4):

- receiving the at least one input previously calculated dose distribution 32 (step 51),
- providing the at least one input previously calculated dose distribution 32 to said augmented radiotherapy generative model 31 so to generate at least one augmented dose distribution 41 with improved quality (step 52),

A particular apparatus may embody the device 1 as well as the device 2 described above. It corresponds for example to a workstation, a laptop, a tablet, a smartphone, or a head-mounted display (HMD).

That apparatus is suited to generation of augmented dose distributions and to related ML training. It comprises the following elements, connected to each other by a bus 95 of addresses and data that also transports a clock signal:

- a microprocessor (or CPU);
- a graphics card comprising several Graphical Processing Units (or GPUs) and a Graphical Random Access Memory (GRAM); the GPUs are quite suited to image processing, due to their highly parallel structure;
- a non-volatile memory of ROM type;
- a RAM;
- one or several I/O (Input/Output) devices such as for example a keyboard, a mouse, a trackball, a webcam; other modes for introduction of commands such as for example vocal recognition are also possible;
- a power source; and
- a radiofrequency unit.

According to a variant, the power supply is external to the apparatus.

The apparatus also comprises a display device of display screen type directly connected to the graphics card to display synthesized images calculated and composed in the graphics card. According to a variant, a display device is external to the apparatus and is connected thereto by a cable or wirelessly for transmitting the display signals. The apparatus, for example through the graphics card, comprises an interface for transmission or connection adapted to transmit a display signal to an external display means such as for example an LCD or plasma screen or a video-projector. In this respect, the RF unit can be used for wireless transmissions.

It is noted that the word "register" used hereinafter in the description of memories can designate in each of the memories mentioned, a memory zone of low capacity (some binary data) as well as a memory zone of large capacity (enabling a whole program to be stored or all or part of the data representative of data calculated or to be displayed). Also, the registers represented for the RAM and the GRAM can be arranged and constituted in any manner, and each of them does not necessarily correspond to adjacent memory locations and can be distributed otherwise (which covers notably the situation in which one register includes several smaller registers).

When switched-on, the microprocessor loads and executes the instructions of the program contained in the RAM.

As will be understood by a skilled person, the presence of the graphics card is not mandatory, and can be replaced with entire CPU processing and/or simpler visualization implementations.

In variant modes, the apparatus may include only the functionalities of the device 1, and not the learning capacities of the device 2. In addition, the device 1 and/or the device 2 may be implemented differently than a standalone software, and an apparatus or set of apparatus comprising only parts of the apparatus may be exploited through an API call or via a cloud interface.

## EXAMPLES

The present invention is further illustrated by the following examples.

Example 1: Training of a U-Net architecture based on dose distributions from VMAT plans

**Materials and Methods**

The dataset comprises around 11000 training samples originating from VMAT plans of 50 patients. The cases in the dataset correspond to various anatomies. The VMAT plans provide a set of high precision 3D dose distributions computed from $1 \times 10^{11}$ particles and a set of low precision 3D dose distributions computed from $1 \times 10^9$ particles. Each training sample is composed of a pair of a high precision 2D dose distribution and a corresponding low precision 2D dose distribution, both corresponding to an axial slice of a high precision 3D dose distribution and of a low precision 3D dose distribution.

The high precision and low precision distributions were normalized using the average dose maximum computed over the high precision 3D dose distributions. To enable batch training, each training sample was padded with zero to match a fixed size of 256x256. To further help the model generate accurate dose predictions, a corresponding CT slice was added as a second input channel to incorporate the corresponding anatomy.

The generative learning model was a U-Net architecture with skip connections between the encoder and the decoder. The encoder part of the model performs downsampling twice with convolutional layers using 4x4 filters and a stride of 2. Symmetrically, transposed convolutions upsample feature maps in the decoder. Each stage of the U-Net comprises two convolution blocks before downsampling or upsampling. A convolution blockfirst applies a convolution with 7x7 filters and 3x3 padding, and then two convolutions with 3x3 filters to further process the feature maps. Each convolution is followed by Gaussian Error Linear Units (GELU) activation units. The block ends with a residual connection to keep high frequency details from the block's input. Overall, the model has around 10 million trainable parameters.

**Optimization set-up:** In all trainings, the model was trained using AdamW optimizer. The initial learning rate was set to $3e^{-4}$ and was decreased progressively during the training when the validation loss stagnated. Weight decay was set to $5e^{-4}$ and batch size to 16. The model was trained for 20000 iterations on a Nvidia GeForce RTX 3090 GPU. The trainings were stopped when overfitting appeared by adopting the early topping strategy. With this training scheme, early stopping occurred after around 15000 iterations, when the validation loss fails to decrease 2% after 500 iterations.

**Annealing schedule:** To train with the GPR loss function $L_{\sigma-GPR}^{\delta}$ and its approximations $L_{\Gamma}^{\delta}(D_r, D_e), L_{\Gamma>1}^{\delta}(D_r, D_e)$ (referred to as GPR-based loss functions), the following annealing schedule for the sharpness parameter β was designed. The initial value of β was set to $2 \times 10^{-2}$ for the first 150 iterations. Then β was increased by a factor of 5% every 50 iterations until it reached an intermediate value of 3, where updates slowed down to 5% every 100 iterations. Increasing updates stopped when β reached a chosen ceiling value of $\beta_{max}$ =5. Setting $\beta_{max}$ prevented the slope of the sigmoid from getting too sharp and the loss from encountering a vanishing gradient problem, which would stop the updates of gradient descent. For all loss functions $L_{\sigma-GPR}^{\delta}$, $L_{\Gamma}^{\delta}(D_r, D_e)$, $L_{\Gamma>1}^{\delta}(D_r, D_e)$, the dose threshold δ was set to 20%. This means that the computed approximated value GPR value takes into account only individual elements Pr for which the dose value is superior to 20% of the maximum dose of the reference distribution, i.e.

$$D_r(P_r) \geq 20\% \cdot \max_{P_r \in D_r} D_r(P_r)$$

**Results**

The performances obtained with the GPR-based loss functions according to the invention were benchmarked against other loss functions used in the computer vision domain. The test set comprised data from 10 patients. Then benchmark includes the MAE (mean absolute error) and the MSE (mean squared error) for a comparison with pixel-wise errors. The combination of pixel-wise errors with the SSIM (structural similarity metric) was also considered. More precisely, the benchmark includes SSIM-MAE and SSIM-MSE, which are the equally weighted sum of respectively the SSIM and MAE, and the SSIM and MSE. The results are summarized in Table 1. For each training on the different loss functions of Table 1, the exact same model architecture and optimization strategy was used, in order to promote the reliability and fairness of the comparison. The metrics were computed for each slice of each patient's volume, and then averaged over the test set per considered metric.

Table 1: Evaluation metrics over the dose distributions comprised in the test set

| Loss function | GPR 2% / 2mm | GPR 2% / 3mm | GPR 3% / 3mm | SSIM (%) | MAE | MSE |
|---|---|---|---|---|---|---|
| MAE | 47.6 ±7.6 | 53.8±8.4 | 66.5±10.0 | 83.3±9.2 | 0.39±0.10 | 0.51±0.28 |
| MSE | 50.2±7.8 | 56.7±8.7 | 69.5±10.3 | 83.9±11.5 | 0.34±0.09 | 0.43±0.29 |
| SSIM+MAE | 46.3±8.8 | 52.3±9.8 | 64.6±11.9 | **94.0±8.8** | 0.35±0.09 | 0.74±0.36 |
| SSIM+MSE | 49.1±10.0 | 55.3±11.1 | 67.5±13.2 | 93.3±3.1 | 0.30±0.07 | 0.30±0.15 |
| $L_{\Gamma}^{\delta}$ | 57.7±3.3 | 65.0±3.5 | 79.2±4.3 | 87.5±7.6 | 0.28±0.07 | 0.30±0.23 |
| $L_{\Gamma>1}^{\delta}$ | 57.2±3.9 | 64.6±4.1 | 79.0±4.4 | 86.7±8.1 | 0.27±0.07 | 0.25±0.16 |
| $L_{\sigma-GPR}^{\delta}$ | **59.3±3.3** | **66.8±3.6** | **81.4±4.0** | **88.2±7.5** | **0.24±0.06** | **0.22±0.16** |

Results point out that models trained with GPR-based loss functions tend to outperform others with respect to the GPR, the MAE and the MSE.

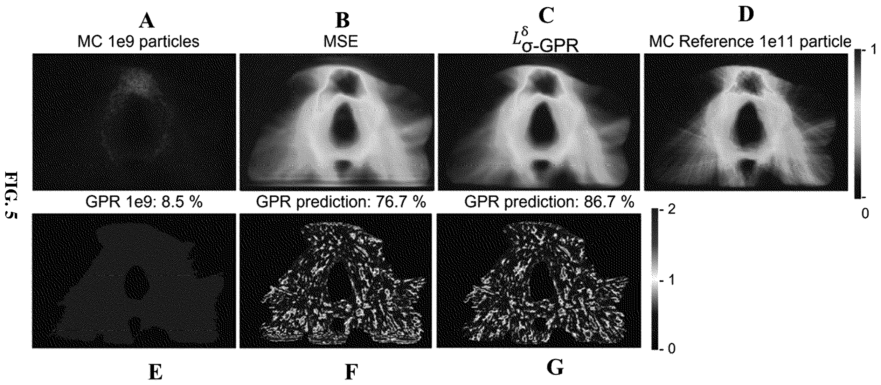

Figure 5 shows, in the first row, single slices corresponding to respectively a $1e^9$ dose volume, a prediction of a model trained with MSE, a prediction of a model trained with $L^{\delta}_{\sigma-GPR}$ and a reference 1e$^{11}$ dose. The second row of Figure 5 shows the gamma index maps for the three different representations.

Example 2: Speed-up of GPR-acceleration approach

**Materials and Methods**

This example illustrates the acceleration embodiments previously described. To quantify the extent of the underlying acceleration approach of those embodiments, two methods were benchmarked. The first is a GPU-accelerated exhaustive search approach in a limited vicinity of 3 mm3 around the considered individual element Pr. The second is an open-source tool from PyMedPhys, which makes use of acceleration ideas from the paper "A fast algorithm for gamma evaluation in 3d", by Wendling M. et al, Medical Physics 34(5) (2007), and executes on CPU ad is single-threaded. For the latter, the interpolation ratio was limited to 2 to have a fair comparison.

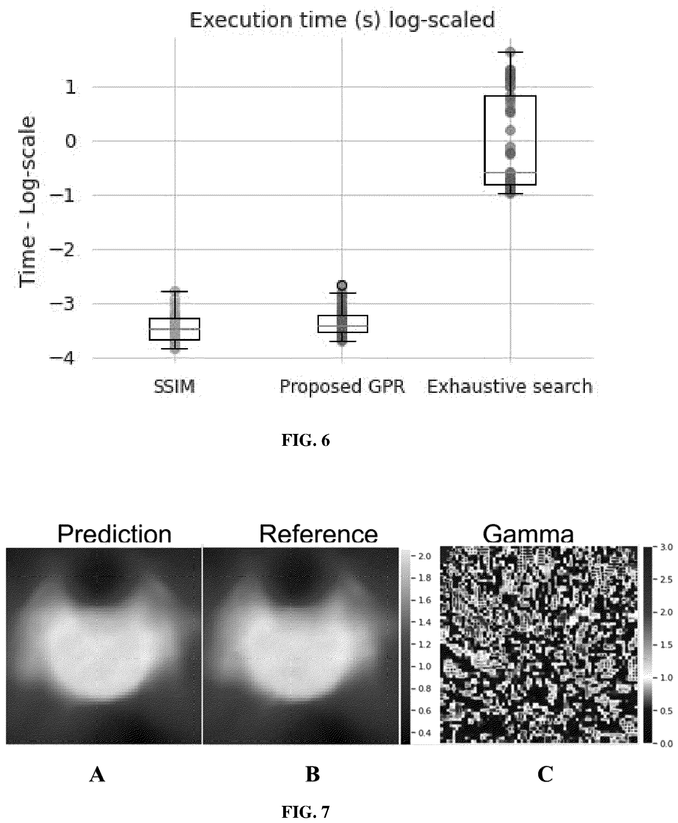

The time estimation was twofold. Each evaluation metric and GPR-based loss functions were timed on 3D or 3D distributions stemming from the dataset used in Example 1. 3D dose distributions were interpolated to resolution 1 mm$^3$ and of shape 128x200x200, comprising around $5 \times 10^6$ voxels. The 2D distributions comprised axial slides of the 3D dose distributions and were interpolated to a size of 400x400. For the GPR calculations, we set the DTA and $\Delta$ to respectively 2 mm and 2%. Execution times are displayed in Table 2. Figure 6 gives another representation of execution times for the SSIM, the acceleration method according to the invention and the exhaustive search method, all on 3D dose distributions.

Table 2: Speed comparison of metrics computed over 2D or 3D dose distributions

| Metric | 3D dose Time (ms) | 2D dose Time (ms) |
|---|---|---|
| MSE | 0.14±0.02 | 0.13±0.03 |
| MAE | 0.23±0.06 | 0.19±0.04 |
| SSIM | 27.49±7.73 | 5.06±3.32 |
| $L^{\delta}_{\sigma-GPR}, L^{\delta}_{\Gamma>1}, L^{\delta}_{\Gamma}$ | **30.54±0.01** | **4.51±0.00** |
| Exhaustive PyMedPhys | 985±515 (> 1 second) | 8.01±2.28 (> 100ms) |

**Results**

Table 2 and Figure 6 highlight that the acceleration approach by using a vicinity of invariant size according to the invention has equivalent speed to that of SSIM. Compared to the exhaustive search method, the acceleration approach according to the invention improves the speed of gamma index computations by a factor of at least 30 in the case of 3D dose distribution and twofold for 2D dose distributions.

Example 3: 3D validation of the training and generating method according to the invention

This example illustrates how the training device according to the invention enables acceleration of computation of high precision dose distributions.

The task presented here aims at translating dose distributions obtained from a Collapse Cone Convolution method (CCC) to augmented dose distributions achieving the precision obtained with a Monte Carlo algorithm. Indeed, CCC resorts to approximations for fast computations of radiotherapy dose distributions. However, approximation impede on accurate dose estimation in areas that contains bone tissue. More precisely, when comparing CCC dose distributions to MC distributions, CCC simulations overestimate the dose in Organs-at-Risk while underestimating the dose deposited in the tumor.

**Dataset:** The cohort comprised 50 patients undergoing radiotherapy for prostate cancer. Corresponding 3D dose distributions were simulated using CCC algorithm and the RadCalc software to compute Monte-Carlo simulations, therefore resulting in 50 paired CCC-MC dose simulations. The resolution of the dose volumes is 2 mm$^3$. Preprocessing consisted in standardizing CT values, and applying minimax normalization on the CCC simulations using the minima and maxima of the MC ground-truth set. Train, validation and test sets comprise respectively 40, 5 and 10 distinct patients.

In this example, the model that was trained based on a 3D GPR-based loss function is a 3D version of the generative Pix2pix model, in order to transform CCC dose distributions into MC dose distributions. The model's parameters were optimized with the Adam algorithm, and a starting learning rate of 0.001 with progressive decay when the validation loss stagnates. Early stopping strategies were used to choose the best performing model.

Results shows that 3D training by using a GPR based- loss function as described in the present disclosure allows qualitatively successful CCC to MC translation of dose distributions as displayed on Figure 7, reaching GPR superior to 95% for some patients. Figure 7 shows indeed a comparison of the dose prediction resulting from the CCC to MC translation with the reference MC dose distribution, as well as the corresponding gamma index map.

**Claims**

1. A device (1) for obtaining a trained augmented radiotherapy generative model (31), configured to generate augmented radiotherapy dose distributions (41) from input, previously calculated, radiotherapy dose distributions (32), said device (1) comprising:

   - at least one input (11) configured to receive a training data set (21) comprising at least one training sample, each training sample comprising at least one radiotherapy dose distribution defined on a domain discretized into a plurality of individual elements
   - at least one processor configured to define said augmented radiotherapy generative model by training an untrained generative model (20) using said training dataset (21) and a GPR loss function,
   wherein said GPR loss function is defined based on a differentiable function approximating a clinical similarity metric, being a Gamma index Passing Rate (GPR), said GPR measuring a similarity between a target radiotherapy dose distribution and a calculated radiotherapy dose distribution,
   - at least one output configured to provide as output said trained augmented radiotherapy generative model (31).

2. The device according to claim **1,** wherein said domain is $\mathbb{R}^k$, with k an integer number equal to 1, 2 or 3.

3. The device according to any one of the preceding claims, wherein said clinical similarity metric is the gamma index passing rate, said gamma index passing rate corresponding to a proportion of said individual elements of said domain satisfying a passing criterion, wherein said passing criterion is based, for each individual element, on a gamma index value.

4. The device according to any one of the preceding claims, wherein said differentiable function is defined by means of a sum of sigmoid functions of sharpness parameter $\beta$.

5. The device according to claim **4,** wherein said at least one processor is further configured to vary, during training, said parameter $\beta$, wherein said parameter $\beta$ is varied by implementing an annealing schedule.

6. The device according to claim **5,** wherein said annealing schedule comprises increasing said parameter $\beta$ during training, and wherein said at least one processor is further configured to, above a given threshold value of said parameter $\beta$, apply a stopgrad operation for individual elements not satisfying said passing criterion.

7. The device according to any one of the preceding claims, wherein the at least one processor is further configured to compute a plurality of gamma index values corresponding to each individual element of said domain, all among the plurality of gamma index values being computed by means of a minimum value search within a vicinity of invariant size.

8. The device according to claim **7,** wherein said invariant size is defined by a distance-to-agreement DTA.

9. The device according to claim **8** and claim **2,** wherein said vicinity is a cube comprising $(2 \times DTA +1)^k$ voxels.

10. The device according to any one of the preceding claims, wherein the at least one generative model is a U-Net.

11. The device according to any one of the preceding claims, wherein each training sample comprises at least two radiotherapy dose distributions, a first reference radiotherapy dose distribution, defined on said domain, and a corresponding second reference radiotherapy dose distribution, also defined on said domain; wherein each first reference radiotherapy dose distribution has higher quality than said corresponding second reference radiotherapy dose distribution.

12. The device according to claim **11,** wherein, for each training sample:

- said first reference radiotherapy dose distribution has been generated with a number of simulated particles having order of magnitude at least one time superior than said corresponding second reference radiotherapy dose distribution, or
- said first reference radiotherapy dose distribution is obtained from a Monte Carlo algorithm and said corresponding second radiotherapy dose distribution is obtained from a collapse-cone convolution algorithm.

13. The device according to any one of claims **1** to **10,** wherein:

- each training sample comprises a first reference radiotherapy dose distribution defined on said domain and a corresponding second reference radiotherapy dose distribution also defined on said domain, wherein each first reference radiotherapy dose distribution and each corresponding second reference radiotherapy dose distribution have low quality;
- said at least one processor is configured to define said augmented radiotherapy generative model by training said untrained generative model using said training dataset and the GPR loss function in a weakly-supervised manner.

14. The device according to any one of claims **1** to **10,** wherein, said at least one processor is configured to define said augmented radiotherapy generative model (31) by training said untrained generative model (20) using said training dataset (21) and the GPR loss function in an unsupervised manner.

15. A device (2) for generating augmented radiotherapy dose distributions (41) from input previously calculated radiotherapy dose distributions (32), using the augmented radiotherapy generative model (31) obtained from the device (1) according to any one of claims **1** to **14,** said device comprising:

- at least one input (15) configured to receive at least one input previously calculated radiotherapy dose distribution (32),
- at least one processor configured to provide said at least one input previously calculated radiotherapy dose distribution (32) to said augmented radiotherapy generative model (31) so as to obtain at least one augmented radiotherapy dose distribution (41),
- at least one output configured to provide said augmented radiotherapy dose distributions (41).

**FIG. 1**

FIG. 2

10

Input previously calculated
dose distributions                    32

Augmented
radiotherapy          31
generative
model

1

Receiving          15

Transforming          17

41          Augmented
dose
distributions

**FIG. 3**

**FIG. 4**

A
MC 1e9 particles

B
MSE

C
$L_\sigma^\delta$-GPR

D
MC Reference 1e11 particle

GPR 1e9: 8.5 %

GPR prediction: 76.7 %

GPR prediction: 86.7 %

E

F

G

FIG. 5

EP 4 478 257 A1

**FIG. 6**

Prediction — **A**     Reference — **B**     Gamma — **C**

**FIG. 7**

Europäisches
Patentamt

European
Patent Office

Office européen
des brevets

# EUROPEAN SEARCH REPORT

Application Number

EP 23 30 5965

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2020/289847 A1 (SJÖLUND JENS OLOF [SE] ET AL) 17 September 2020 (2020-09-17) * paragraphs [0005] - [0010], [0018] - [0087]; figures 2-6 * | 1-15 | INV. G06N3/088 G06N3/0895 G06N20/00 A61N5/10 |
| A | US 2021/192719 A1 (LAAKSONEN HANNU MIKAEL [FI] ET AL) 24 June 2021 (2021-06-24) * paragraphs [0002] - [0003], [0021] - [0080]; figures 1-4 * | 1-15 | |
| A | US 2020/075148 A1 (NGUYEN DAN [US] ET AL) 5 March 2020 (2020-03-05) * paragraphs [0004] - [0007], [0117] - [0130], [0210] - [0275]; figures 1A-C, 2-3 * | 1-15 | |
| A | US 2023/154586 A1 (CASTLE JAMES [US] ET AL) 18 May 2023 (2023-05-18) * paragraphs [0006] - [0009], [0077] - [0089]; figures 1-3 * | 1-15 | |

TECHNICAL FIELDS
SEARCHED (IPC)

G06N
A61N

The present search report has been drawn up for all claims

| Place of search | Date of completion of the search | Examiner |
|---|---|---|
| Munich | 28 November 2023 | Lahorte, Philippe |

EPO FORM 1503 03.82 (P04C01)

## ANNEX TO THE EUROPEAN SEARCH REPORT
## ON EUROPEAN PATENT APPLICATION NO.

**EP 23 30 5965**

This annex lists the patent family members relating to the patent documents cited in the above-mentioned European search report.
The members are as contained in the European Patent Office EDP file on
The European Patent Office is in no way liable for these particulars which are merely given for the purpose of information.

**28-11-2023**

| Patent document cited in search report | Publication date | Patent family member(s) | | Publication date |
|---|---|---|---|---|
| US 2020289847 A1 | 17-09-2020 | NONE | | |
| US 2021192719 A1 | 24-06-2021 | CN | 114846476 A | 02-08-2022 |
| | | EP | 4078457 A1 | 26-10-2022 |
| | | US | 2021192719 A1 | 24-06-2021 |
| | | WO | 2021122615 A1 | 24-06-2021 |
| US 2020075148 A1 | 05-03-2020 | US | 2020075148 A1 | 05-03-2020 |
| | | US | 2023352135 A1 | 02-11-2023 |
| | | WO | 2020047536 A1 | 05-03-2020 |
| US 2023154586 A1 | 18-05-2023 | NONE | | |

EPO FORM P0459

For more details about this annex : see Official Journal of the European Patent Office, No. 12/82

**REFERENCES CITED IN THE DESCRIPTION**

**Non-patent literature cited in the description**

- **WENDLING M. et al.** A fast algorithm for gamma evaluation in 3d. *Medical Physics*, 2007, vol. 34 (5)